# EUROPEAN PATENT APPLICATION

(11) **EP 3 845 066 A1**
(43) Date of publication of application: **07.07.2021**
(21) Application number: 20216828.2
(22) Date of filing: 23.12.2020
(51) Int. Cl.: A01N 43/16, A01N 25/02, A01N 25/34, A01P 1/00, A41D 19/00, A41D 31/30, A61B 42/00

(54) **AN ANTIMICROBIAL ELASTOMERIC FORMULATION**

(30) Priority: 30.12.2019 MY PI2019007877
(71) Applicant: TOP GLOVE INTERNATIONAL SDN. BHD., 41050 Klang Selangor (MY)
(72) Inventor: WONG, Chong Ban, 41050 Klang (MY); LIM, Keuw Wei, 41050 Klang (MY); P.CHANDRASAKARAN, Devi Shantini, 41050 Klang (MY)
(74) Representative: Schiweck Weinzierl Koch Patentanwälte Partnerschaft mbB

(57) **Abstract**

The present invention relates to an antimicrobial coagulant solution, wherein the antimicrobial coagulant solution is a mixture of an antimicrobial additive and a coagulant solution. The antimicrobial additive formulation comprises (i) natural ingredient, (ii) organic acid and (iii) water. The coagulant solution comprises (i) coagulating agent, (ii) antitack agent, (iii) wetting agent, (iv) pH adjuster and (v) solvent. Further, present invention discloses an antimicrobial elastomeric article comprising antimicrobial coating that is prepared from the antimicrobial coagulant solution. The antimicrobial elastomeric article is an antimicrobial glove.

## Description

### FIELD OF THE INVENTION

The present invention relates to an antimicrobial elastomeric article, wherein the antimicrobial elastomeric article comprises antimicrobial coating. The antimicrobial coating is prepared from an antimicrobial coagulant solution which comprises an antimicrobial additive that is derived from natural ingredient.

### BACKGROUND OF THE INVENTION

The growth and proliferation of microorganisms generally occur because of exposure to prolonged warm and moist environments. Microorganisms can easily proliferate on the object that is in contact with the environment. Thus, antimicrobial agents have been incorporated into many different substrates for preventing and controlling the growth of microorganisms.

Further, due to growing public health awareness on pathogenic effects, intensive research and development have been promoted in order to minimize or even eliminate microorganism's growth on various products in particular gloves and personal care products. The microbial contamination is a great concern, mainly for gloves used in hospitals as personal protective equipment. The infections acquired in hospitals are mostly caused by several species, such as *Escherichia coli, Pseudomonas aeruginosa, Klebsiella pneumoniae, Staphylococcus aureus, Enterococcus faecalis, Streptococcus pyogenes and* Methicillin resistance *staphylococcus aureus* (MRSA).

Therefore, the demand for antimicrobial glove has increased over the years as consumers are becoming increasingly aware of the implications on personal hygiene and the health risks associated with certain microorganisms.

Chemicals are used as antimicrobial additive for the past attempts to develop antimicrobial elastomer products in particular antimicrobial glove. However, the long-term usage of chemicals may lead to unforeseen side effects. Thus, there is a need to substitute the chemicals with natural ingredient and a need to identify a formulation which uses the antimicrobial additive that is derived from natural ingredient as to overcome the above setback.

### SUMMARY OF THE INVENTION

The present invention relates to an antimicrobial coagulant solution (ACS). The antimicrobial coagulant solution is a mixture of an antimicrobial additive (AD) and a coagulant solution (CS). The antimicrobial additive AD comprises (i) natural ingredient in an amount ranging between 1.00% to 2.00% by w/v, (ii) organic acid in an amount ranging between 0.50% to 2.00% by v/v and (iii) water used in an (remaining) amount to achieve 100% by weight of the antimicrobial additive. The coagulant solution CS comprises (i) a coagulating agent in an amount ranging between 8.00% to 10.00% by w/w, (ii) an antitack agent in an amount ranging between 1.50% to 2.00% by w/w, (iii) a wetting agent in an amount ranging between 0.08% to 0.50% by w/w, (iv) a pH adjuster in an amount ranging between 0.002% to 0.006% by w/w and (v) solvent used in an amount to achieve 100% by weight of the coagulant solution CS. Further, the present invention relates to an antimicrobial elastomeric article that comprises an antimicrobial coating that is prepared from the antimicrobial coagulant solution (ACS) of the invention. The antimicrobial elastomeric article is preferably an antimicrobial glove.

### BRIEF DESCRIPTION OF THE ACCOMPANYING DRAWINGS

The present invention will be fully understood from the detailed description given herein below and the accompanying drawing which are given by way of illustration only, and thus are not limitative of the present invention, wherein:
In the appended drawing:

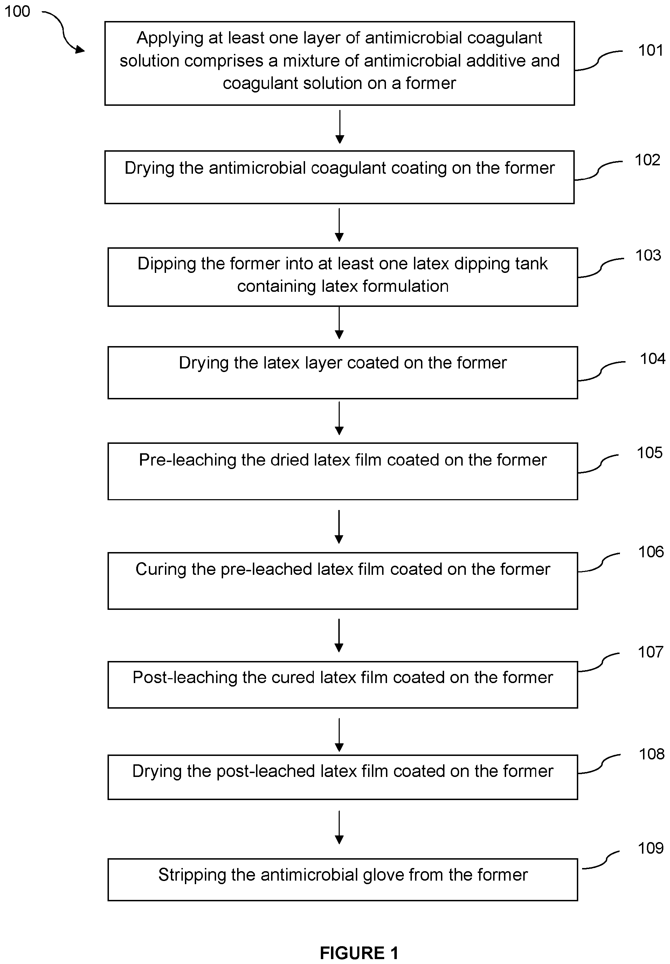

**FIGURE 1** is a diagram showing the method of producing an antimicrobial glove in accordance with an embodiment of the present invention.

### DETAILED DESCRIPTION OF THE INVENTION

Detailed description of preferred embodiments of the present invention is disclosed herein. It should be understood, however, that the embodiments are merely exemplary of the present invention, which may be embodied in various forms. Therefore, the details disclosed herein are not to be interpreted as limiting, but merely as the basis for the claims and for teaching one skilled in the art of the invention. The numerical data or ranges used in the specification are not to be construed as limiting. The following detailed description of the preferred embodiments will now be described in accordance with the attached drawings.

The present invention relates to an antimicrobial elastomeric article, wherein the antimicrobial elastomeric article comprises an antimicrobial coating. The antimicrobial coating is prepared from an antimicrobial coagulant solution as disclosed herein which comprises antimicrobial additive that is derived from natural ingredient. The natural ingredient is chitosan. The antimicrobial elastomeric article may, for example be an antimicrobial glove, an antimicrobial condom, a antimicrobial dental dam, or an antimicrobial finger cot.

For the purpose of the present invention, the term "coating" is related to a formation of a layer of film on a glove former at multiple stages during the manufacturing process to yield a finished glove product. The finished glove product is formed from more than one layer of coating, for instance, coagulant coating, latex coating and so on.

First aspect of the present invention relates to an antimicrobial coagulant solution wherein the antimicrobial coagulant solution is a mixture of an antimicrobial additive and a coagulant solution.

For the purpose of the present invention, the term "antimicrobial additive" signifies the antimicrobial property exhibited by natural ingredient which is able to kill or suppress the growth of microorganisms.

The antimicrobial additive AD comprises (i) natural ingredient, (ii) organic acid and (iii) water used in an amount to achieve 100% by weight of the antimicrobial additive. The natural ingredient is chitosan. The natural ingredient is used in an amount ranging between 1.00% to 2.00% by w/v, preferably 1.10% to 1.50% by w/v, still preferably 1.20% by w/v of the total weight of the antimicrobial additive AD. The organic acid is any one or more selected from a group consisting of acetic acid, hydrochloric acid or combinations thereof, preferably acetic acid. The organic acid is used in an amount ranging between 0.50% to 2.00% by v/v, preferably 0.70% to 1.90% by v/v, still preferably 1.80% by v/v of the total weight of the antimicrobial additive AD.

Table 1 shows formulation of the antimicrobial additive.

**Table 1: Formulation of the antimicrobial additive**

| **Materials** | **Working range (%)** | **Preferred range (%)** | **Typical value (%)** |
|---|---|---|---|
| Natural ingredient (w/v) | 1.00 to 2.00 | 1.10 to 1.50 | 1.20 |
| Organic acid (v/v) | 0.50 to 2.00 | 0.70 to 1.90 | 1.80 |
| Water | Used in an amount to achieve 100% by weight of the antimicrobial additive | | |

The antimicrobial additive prepared in accordance to Table 1 is added in an amount ranging between 0.10% to 0.40% by w/w, preferably 0.15% to 0.35% by w/w, still preferably 0.20% by w/w into a coagulant solution to prepare the antimicrobial coagulant solution. The amount of antimicrobial additive is prepared based on the amount of coagulant solution wherein the ratio of natural ingredient (w/v) to organic acid (v/v) is 2:3.

The coagulant solution CS comprises (i) a coagulating agent, (ii) an antitack agent, (iii) a wetting agent, (iv) a pH adjuster and (v) solvent.

The coagulating agent is any one or more selected from group consisting of calcium nitrate, calcium chloride and any combinations thereof, preferably calcium nitrate. The coagulating agent is used in an amount ranging between 8.00% to 10.00% by w/w, preferably 8.10% to 9.90% by w/w, still preferably 8.90% by w/w of the total weight of the coagulant solution CS.

The antitack agent is any one more selected from group consisting of potassium stearate, calcium stearate and any combinations thereof, preferably potassium stearate. The antitack agent is used in an amount ranging between 1.50% to 2.00% by w/w, preferably 1.60% to 1.90% by w/w, still preferably 1.80% by w/w of the total weight of the coagulant solution CS.

The wetting agent is any one or more selected from a group consisting of nonionic surfactant or anionic surfactant. Nonionic surfactant is any one or more selected from the group consisting of alcohol ethoxylates such as isotridecanol ethoxylates, ethylene oxide or combinations thereof. Anionic surfactant is any one or more selected from the group consisting of disodium N-octadecylsulphosuccinamate, sodium dihexyl sulfosuccinate, sodium dioctyl sulfosuccinate or combinations thereof. The wetting agent is preferably isotridecanol ethoxylates. The wetting agent is used in an amount ranging between 0.08% to 0.50% by w/w, preferably 0.10% to 0.45% by w/w, still preferably 0.12% by w/w of the total weight of the coagulant solution CS. The wetting agent functions to improve uniform wetting on the former for better adhesion of latex layer on coagulant coating.

The pH adjuster is any one or more selected from the group consisting of ammonia, potassium hydroxide, sodium hydroxide and ammonium hydroxide, preferably ammonia. The pH adjuster is used in an amount ranging between 0.002% to 0.006% by w/w, preferably 0.003% to 0.005% by w/w, still preferably 0.004% by w/w of the total weight of the coagulant solution CS. The pH adjuster functions to adjust the alkalinity of the coagulant for even adhesion of latex layer on coagulant coating.

The solvent is any one selected from the group consisting of tap water, distilled water, deionized water and any combinations thereof, preferably tap water. The solvent is used in an amount to achieve 100% by weight of the coagulant solution.

Table 2 shows formulation of the coagulant solution.

**Table 2: Formulation of the coagulant solution**

| **Materials** | **Working range (%)** | **Preferred range (%)** | **Typical value (%)** |
|---|---|---|---|
| Coagulating agent (w/w) | 8.00 to 10.00 | 8.10 to 9.90 | 8.90 |
| Antitack agent (w/w) | 1.50 to 2.00 | 1.60 to 1.90 | 1.80 |
| Wetting agent (w/w) | 0.08 to 0.50 | 0.10 to 0.45 | 0.12 |
| pH adjuster (w/w) | 0.002 to 0.006 | 0.003 to 0.005 | 0.004 |
| Solvent | Used in an amount to achieve 100% by weight of the coagulant solution | | |

Second aspect of the present invention discusses on the antimicrobial glove of present invention. The antimicrobial glove of the present invention includes at least one layer of coating, wherein the coating is antimicrobial coating. The antimicrobial coating is prepared from the above-mentioned antimicrobial coagulant solution.

The antimicrobial glove of the present invention is prepared adopting the commonly known method in the glove manufacturing industry. The antimicrobial glove of the present invention included chlorinated antimicrobial glove, polymer coated antimicrobial glove, powdered antimicrobial glove but not limited thereto.

**FIGURE 1** shows a flow diagram of the method for producing antimicrobial glove in accordance with the exemplary embodiment of the present invention.

A method to produce an antimicrobial glove (10) comprises the steps of:
(i) applying at least one layer of antimicrobial coagulant solution (as described in first aspect) on a former at a temperature ranging between 55°C to 65°C for a time period ranging between 10 seconds to 15 seconds to produce an antimicrobial coagulant coating on the former, wherein the coating may be applied by way of dipping the former into a dipping tank containing the antimicrobial coagulant solution or spraying the antimicrobial coagulant solution onto the former, wherein the coating may be carried out offline and/or online (101);
(ii) drying the antimicrobial coagulant coating on the former obtained in step (i) in an oven at a temperature ranging between 130°C to 140°C for a time period ranging between 2 minutes to 3 minutes (102);
(iii) dipping the former obtained in step (ii) into at least one latex dipping tank containing latex formulation at a temperature ranging between 50°C to 65°C for a time period ranging between 1 minute to 2 minutes to produce a latex layer coated on the former wherein the latex formulation comprises at least a base polymer, a dispersing agent, an activator, a pH adjuster, a crosslinker, an accelerator, a wetting agent and an antifoaming agent (103);
(iv) drying the latex layer coated on the former obtained in step (iii) at a temperature ranging between 50°C to 70°C for a time period ranging between 1 minute to 2 minutes to obtain dried latex film (104);
(v) pre-leaching the dried latex film coated on the former obtained in step (iv) with hot water at a temperature ranging between 60°C to 70°C for a time period ranging between 1 minute to 2 minutes to leach out excess chemical residues to obtain pre-leached latex film (105);
(vi) curing the pre-leached latex film coated on the former obtained in step (v) in an oven at a temperature ranging between 115°C to 140°C for a time period ranging between 12 minutes to 15 minutes to produce cured latex film (106);
(vii) post-leaching the cured latex film coated on the former obtained in step (vi) using hot water at a temperature ranging between 60°C to 65°C for a time period ranging between 0.5 minute to 1.5 minutes to leach out excess chemical residues to obtain post-leached latex film (107);
(viii) drying the post-leached latex film coated on the former in step (vii) in an oven at a temperature ranging between 100°C to 115°C for a time period ranging between 1 minute to 2 minutes to produce antimicrobial glove (108); and
(ix) stripping the antimicrobial glove obtained in step (ix) from the former (109).

The above method discusses on producing antimicrobial glove through online process in which the antimicrobial coagulant solution is applied during online dipping process but it is not limited thereto.
The control glove and antimicrobial glove of the present invention were subjected to an antimicrobial efficacy test. The antimicrobial properties of the gloves were studied against seven types of bacteria which are *Escherichia coli, Pseudomonas aeruginosa, Klebsiella pneumoniae, Staphylococcus aureus, Enterococcus faecalis, Streptococcus pyogenes and* Methicillin resistance *staphylococcus aureus* (MRSA). The antimicrobial efficacy is measured based on ASTM D7907 standard test method. The results obtained are tabulated in Table 3.

**Table 3: Antimicrobial efficacy of control glove and antimicrobial glove of the present invention**

| **Bacteria** | **Type** | **Control glove** | | | | | **Antimicrobial glove of present invention** | | | | |
|---|---|---|---|---|---|---|---|---|---|---|---|
| | | **0 min** | **5 min** | **10 min** | **20 min** | **30 min** | **0 min** | **5 min** | **10 min** | **20 min** | **30 min** |
| *Escherichia coli* | Gram- negative | 7.55 | 20.75 | 26.42 | 32.08 | 35.85 | 24.53 | 28.30 | 33.96 | 54.72 | 69.81 |
| *Pseudomonas aeruginosa* | | 22.64 | 22.64 | 30.19 | 35.85 | 39.62 | 79.25 | 86.23 | 88.11 | 90.38 | 92.26 |
| *Klebsiella pneumoniae* | | 11.90 | 23.81 | 23.81 | 26.19 | 28.57 | 16.67 | 33.33 | 52.58 | 79.29 | 95.71 |
| *Staphylococcus aureus* | Gram- positive | 15.63 | 25.00 | 31.35 | 34.38 | 37.80 | 31.25 | 62.50 | 78.13 | 92.50 | 97.09 |
| *Enterococcus faecalis* | | 9.09 | 22.27 | 31.82 | 40.91 | 40.91 | 54.55 | 60.45 | 66.82 | 75.00 | 80.91 |
| *Streptococcus pyogenes* | | 5.00 | 17.50 | 22.50 | 27.50 | 30.00 | 37.50 | 75.25 | 84.50 | 88.25 | 93.00 |
| Methicillin resistance *staphylococcus aureus* (MRSA) | | 10.34 | 20.69 | 27.59 | 37.93 | 41.38 | 31.03 | 41.38 | 68.27 | 69.66 | 75.86 |

The results obtained showed that the antimicrobial glove of present invention was able to kill all seven types of bacteria which are *Escherichia coli, Pseudomonas aeruginosa, Klebsiella pneumoniae, Staphylococcus aureus, Enterococcus faecalis, Streptococcus pyogenes and* Methicillin resistance *staphylococcus aureus* (MRSA). The antimicrobial efficacy of control glove is significantly lower than the antimicrobial glove of the present invention. Further, the antimicrobial efficacy is increased when the duration of the antimicrobial glove being exposed to the bacteria is increased.

Additionally, both coagulant solution without antimicrobial additive and coagulant solution with antimicrobial additive were tested for wetting properties by measuring contact angle. The results obtained are shown in Table 4.

**Table 4: Wetting properties of coagulant solution without antimicrobial additive and coagulant solution with antimicrobial additive**

| **Type of coagulant** | **Contact angle (°)** | |
|---|---|---|
| | **Standard range** | **Typical value** |
| Coagulant solution without antimicrobial additive | 30 to 33 | 32.06 |
| Coagulant solution with antimicrobial additive | 30 to 33 | 31.00 |

The results obtained showed that coagulant solution with antimicrobial additive of present invention has lower contact angle which indicates that it has better wetting properties for covering the former surface as a layer than coagulant solution without antimicrobial additive.

The antimicrobial glove of the present invention may be used in variety of applications such as but not limited to cleanroom, food handling, cosmetic, biomedical, semiconductor, electrical and/or healthcare. Additionally, teachings of the present invention are not limited to gloves as person skilled in the art can adopt the teachings of present invention for any other elastomeric articles which exhibit similar elastomeric characteristics such as dental dams, condoms, finger cots and the like.

As a whole, the antimicrobial glove of the present invention is able to overcome the conventional glove's shortcomings since the antimicrobial glove of the present invention which comprises antimicrobial additive is able to utilize natural ingredient as antimicrobial additive. Further, the wetting properties of coagulant solution with antimicrobial additive of present invention also showed that the coagulant solution can cover the former surface easily and evenly. Thus, the antimicrobial coagulant solution of present invention that uses natural ingredient as antimicrobial additive is able to eliminate the use of chemicals as antimicrobial additive.

The terminology used herein is for the purpose of describing particular example embodiments only and is not intended to be limiting. As used herein, the singular forms "a", "an" and "the" may be intended to include the plural forms as well, unless the context clearly indicates otherwise. The terms "comprises", "comprising", "including" and "having" are inclusive and therefore specify the presence of stated features, integers, steps, operations, elements and/or components, but do not preclude the presence or addition of one or more other features, integers, steps, operations, elements, components and/or groups therefrom.

The method, steps, processes and operations described herein are not to be construed as necessarily requiring their performance in the particular order discussed or illustrated, unless specifically identified as an order of performance. It is also to be understood that additional or alternative steps may be employed. The use of the expression "at least" or "at least one" suggests the use of one or more elements, as the use may be in one of the embodiments to achieve one or more of the desired objects or results.

## Claims

1. An antimicrobial coagulant solution which is a mixture of an antimicrobial additive AD and a coagulant solution CS,
wherein a) the antimicrobial additive AD comprises (i) a natural ingredient, (ii) an organic acid and (iii) water used in an amount to achieve 100% by weight of the antimicrobial additive, wherein the natural ingredient is chitosan and the organic acid is any one or more selected from the group consisting of acetic acid, hydrochloric acid or combinations thereof, and wherein b) the coagulant solution CS comprises (i) a coagulating agent, (ii) an antitack agent, (iii) a wetting agent, (iv) a pH adjuster and (v) solvent.

2. The antimicrobial coagulant solution as claimed in claim 1, wherein the natural ingredient is used in an amount ranging between 1.00% to 2.00% by w/v of the total weight of the antimicrobial additive AD.

3. The antimicrobial coagulant solution as claimed in claim 1 or 2, wherein the organic acid is used in an amount ranging between 0.50% to 2.00% by v/v of the total weight of the antimicrobial additive AD.

4. The antimicrobial coagulant solution as claimed in any of claims 1 to 3, wherein the coagulating agent is any one or more selected from group consisting of calcium nitrate, calcium chloride and any combinations thereof.

5. The antimicrobial coagulant solution as claimed in any of claims 1 to 4, wherein the coagulating agent is used in an amount ranging between 8.00% to 10.00% by w/w of the total weight of the coagulant solution CS.

6. The antimicrobial coagulant solution as claimed in any of claims 1 to 5, wherein the antitack agent is any one more selected from group consisting of potassium stearate, calcium stearate and any combinations thereof.

7. The antimicrobial coagulant solution as claimed in any of claims 1 to 6, wherein the antitack agent is used in an amount ranging between 1.50% to 2.00% by w/w of the total weight of the coagulant solution CS.

8. The antimicrobial coagulant solution as claimed in any of claims 1 to 7, wherein the wetting agent is any one or more selected from a group consisting of nonionic surfactant or anionic surfactant, wherein the nonionic surfactant is any one or more selected from the group consisting of alcohol ethoxylates, wherein the alcohol ethoxylates is isotridecanol ethoxylates, ethylene oxide or combinations thereof and anionic surfactant is any one or more selected from the group consisting of disodium N-octadecylsulphosuccinamate, sodium dihexyl sulfosuccinate, sodium dioctyl sulfosuccinate or combinations thereof.

9. The antimicrobial coagulant solution as claimed in any of claims 1 to 8, wherein the wetting agent is used in an amount ranging between 0.08% to 0.50% by w/w of the total weight of the coagulant solution CS.

10. The antimicrobial coagulant solution as claimed in any of claims 1 to 9, wherein the pH adjuster is any one selected from the group consisting of ammonia, potassium hydroxide, sodium hydroxide and ammonium hydroxide.

11. The antimicrobial coagulant solution as claimed in any of claims 1 to 10, wherein the pH adjuster is used in an amount ranging between 0.002% to 0.006% by w/w of the total weight of the coagulant solution CS.

12. The antimicrobial coagulant solution as claimed in any of claims 1 to 11, wherein the solvent is any one selected from the group consisting of tap water, distilled water, deionized water and any combinations thereof.

13. The antimicrobial coagulant solution as claimed in any of claims 1 to 12, wherein the solvent used in an amount to achieve 100% by weight of the coagulant solution.

14. The antimicrobial coagulant solution as claimed in any of claims 1 to 13, wherein the antimicrobial additive AD is added in a range of 0.1% to 0.4% by w/w of the total weight of the antimicrobial coagulant solution into the coagulant solution CS to prepare an antimicrobial coagulant solution.

15. An antimicrobial elastomeric article that includes at least one layer of coating, wherein the coating is an antimicrobial coating and wherein the antimicrobial coating is prepared from the antimicrobial coagulant solution as claimed in claims 1 to 14, wherein the antimicrobial elastomeric article is preferably an antimicrobial glove.
